# EUROPÄISCHE PATENTANMELDUNG

(11) **EP 1 287 848 A2**
(43) Veröffentlichungstag der Anmeldung: **05.03.2003**
(21) Anmeldenummer: 02018775.3
(22) Anmeldetag: 22.08.2002
(51) Int. Cl.: A61N 1/16, A61N 2/06

(54) **Magnetfeld-Vorrichtung mit integrierter Abschirmung, insbesondere als Matratzenauflage**

(30) Priorität: 25.08.2001 DE 10141673
(71) Anmelder: SAMINA Produktions- und Handels GmbH, 6820 Frastanz (AT)
(72) Erfinder: Amann, Günther W., 6820 Frastanz (AT)
(74) Vertreter: Riebling, Peter, Dr.-Ing.

(57) **Zusammenfassung**

Die Erfindung betrifft eine Magnetfeld-Vorrichtung, welche mindestens einen, bevorzugt jedoch eine Vielzahl von untereinander beabstandeten, Permanentmagneten mit Nord- und Südpol enthält und mindestens eine, mindestens teilweise elektrisch leitfähige, jedoch nicht magnetisierbare und elektrisch erdbare Schicht zur Abschirmung unerwünschter fremder Energiefelder enthält, auf welcher die Permanentmagnete in Folienform aufgebracht sind. Umhüllt wird diese Anordnung von einem wärmedämmenden Schafschurwoll-Vlies und einem äußeren Baumwollstoff. Durch Abschirmung schädlicher, fremder Energiefelder und Einkopplung von erwünschten, heilsamen Magnetfeldern in den Körper, die dem natürlichen Erdmagnetfeld in gesteigerter Form entsprechen, wird ein prophylaktischer und therapeutischer Effekt erzielt, der das Schlafverhalten, die Schlafqualität sowie das energetische Gleichgewicht der Körperzellen positiv beeinflussen kann.

## Beschreibung

Die vorliegende Erfindung betrifft eine Magnetfeld-Vorrichtung mit integrierter Abschirmung und insbesondere eine Matratzenauflage für menschliche Körper nach dem Oberbegriff des unabhängigen Anspruches. Durch die Erfindung sollen aber nicht nur Matratzenauflagen erfasst sein, sondern auch Sitzkissen, insbesondere für Kfz-Sitze, Schlafdecken oder aber auch Bekleidungsstücke. Auch ist die Anwendung der Erfindung nicht nur für Menschen vorgesehen, sondern natürlich auch für Tiere oder gar Pflanzen.

Im folgenden wird nur noch Bezug genommen auf Magnetfeld-Vorrichtungen in Form von einer Matratzenauflage oder einem Sitzkissen für den Menschen, was für den Schutzbereich der vorliegenden Erfindung jedoch nicht als einschränkend aufzufassen ist.

Die vorliegende Erfindung, nämlich insbesondere in Gestalt einer Magnetfeld-Matratzenauflage, ist ein Ergebnis der Energie- und Informationsmedizin-Forschung. Das Ziel ist die Verbesserung des Schlafverhaltens und der Schlafqualität sowie die Aufrechterhaltung/Wiederherstellung des energetischen Gleichgewichtes der Körperzellen. Dadurch sollen prophylaktisch Gesundheitsstörungen vermieden und im Bedarfsfalle der Körper/Organismus über Selbstheilungsmechanismen positiv beeinflusst werden, vorzugsweise während der Schlafphase.

Mit dem Gegenstand der DE 93 01 830 U1 ist ein Magnetkissen mit eingebauten Permanentmagneten bekannt geworden, bei denen die Wirkung des Permanent-Magnetfeldes durch den Einbau einer magnetisch leitfähigen Platte verstärkt werden soll, die parallel zur Matratzenebene ausgerichtet ist. Es ist jedoch bekannt, dass dieses Permanent-Magnetfeld durch hochfrequente Störfelder beeinflussbar und dadurch stark gestört wird. Derartige HF-Felder werden durch die modernen Telekommunikatiosgeräte (Handys) bewirkt und auch duch die Sendestrahlen der ständig sendenden Basisstationen. Ferner durch Funkfrequenzen der Zeitsignalsender, durch Elektrosmog und andere Störfelder. Vorkehrungen, die Ausbildung eines ungestörten Permanent-Magnetfeldes zu ermöglichen, sind aus dieser Druckschrift nicht zu entnehmen.

Die DE 8518370 U1, DE 4136374 C2, DE 83 08 699 U1, DE 88 10 663 U1, DE 91 09 270 U1, DE 4004682 A1, DE 35 22 667 A1, DE 3336797 A1 lehren lediglich die Erzeugung von Magnetfeldern in einer Auflage, wobei die Magnetfelder entweder durch Permanentmagnete oder durch fremderregte Induktionsspulen erzeugt werden. Auch dort fehlen Vorkehrungen zur Abschirmung der störenden HF-Felder.

Der Erfindung liegt deshalb ausgehend von der DE 33 36 797 A1 die Aufgabe zugrunde, eine Auflage oder Unterlage, insbesondere eine Matratzenauflage der eingangs genannten Art so weiterzubilden, dass die Ausbreitung und Einwirkung eines Magntfeldes auf den menschlichen Körper nicht durch hochfrequente, eletromagnetische Felder gestört wird.

Zur Lösung der gestellten Aufgabe dient die technische Lehre des unabhängigen Anspruches. Vorteilhafte Weiterbildungen der Erfindung sind in den darauf bezogenen Unteransprüchen angeführt.

Durch das erfindungsgemäße Magnetfeldunterbett kann und soll der Schlafplatz therapeutisch genutzt werden. Grundlage dafür ist die energetische Harmonisierung und Aufladung sämtlicher Körperzellen durch die Einwirkung biologisch relevanter, statischer Magnetfelder. Einerseits sollen durch die Erfindung gesundheitsförderliche natürliche Felder, wie das Erdmagnetfeld, erhalten und verstärkt werden, andererseits sollen unerwünschte Feldeinflüsse, wie technische Felder, oder unerwünschte natürliche Feldeinflüsse, wie geo-biologische Felder, abgeschirmt und/oder neutralisiert werden.

Vorteil der vorliegenden Erfindung ist es, dass einerseits das Erdmagnetfeld in Homogenität und Intensität verstärkt und stabilisiert wird sowie bestehende Überlagerungen, hervorgerufen durch magnetisierte Bau- und Einrichtungsmaterialien, ausgeglichen werden und gleichzeitig die 50-Hz-Wechselfelder sowie elektrostatische Aufladungen vom Körper ferngehalten bzw. abgeleitet werden. Zusätzlich können die heilenden Kräfte durch permanente und statische Magnetfelder während des Schlafes genutzt werden.

Dadurch soll der Schlafplatz im Sinne einer "passiven Therapie" in Form einer natürlichen Magnetfeld-Therapie zum Zwecke der Verbesserung des Schlafverhaltens und zur Aktivierung des Immunsystems genutzt werden.

Ein entscheidender Vorteil der vorliegenden Erfindung ist es, dass durch die Abschirmung und Ableitung fremder künstlicher Energiefelder, insbesondere der elektrischen 50-Hz-Wechselfelder und elektrostatischer Aufladungen, der Organismus besser in der Lage ist, die heilsamen Magnetfelder aufzunehmen.

Die Erfindung soll auch der Tatsache Rechnung tragen, dass es nur in Ausnahmefällen möglich ist, eine Schlafstelle vorteilhaft so einzurichten, dass der Kopf im Norden und die Füße im Süden zu liegen kommen, um die Feldkräfte des natürlichen Erdmagnetfeldes optimal ausnutzen zu können. Durch die Verwendung und Anordnung natürlich wirkender Permanentmagnet-Segmente wird erreicht, dass das natürliche Erdmagnetfeld so verstärkt wird, dass durch die entsprechende Polarisation der Magnetfeld-Matratzenauflage der Kopf immer in Nordrichtung und die Füße immer in Südrichtung des verstärkten Erdmagnetfeldes zu liegen kommen.

Die Erfindung berücksichtigt auch, dass die Aufnahme bzw. Wirkung von therapeutischen Magnetfeldern ebenfalls durch geo-biologische Einflüsse stark gemindert werden kann. Durch die Verwendung eines Spezialgewebes aus einer Baumwoll-Silber-Mischung erfolgt eine gleichzeitige Abschirmung/Neutraiisation dieser unerwünschten Feldeinflüsse innerhalb der Liegefläche.

Gleichzeitig sollen auch alle bekannten, wissenschaftlich relevanten Vorteile und Möglichkeiten einer Magnetfeldtherapie mit ruhenden Magnetfeldern genutzt werden. Bei Permanentmagneten handelt es sich um statische Magnetfelder, wie beim natürlichen Magnetfeld der Erde. Während mit elektrischem Strom betriebene Magnetfeld-Behandlungsgeräte ein elektromagnetisches Feld aufbauen, geht von der vorliegenden Magnetfeld-Matratzenauflage keine Strahlung aus. Magnetfelder durchdringen alle organischen Stoffe. Nebenwirkungen sind nicht bekannt. Thermische Nebenreaktionen treten nicht auf. Besondere Vorbereitungen für die Anwendung sind nicht erforderlich, da die Magnetfeld-Matratzenauflage ein fixer Bestandteil der Schlafunterlage ist. Die über die spezielle Matratzenauflage ermöglichte Magnetfeldtherapie lässt sich mit anderen Therapiearten hervorragend kombinieren und ergänzen.

Klinischen Tests der zur Anwendung gelangenden und verwendeten Permanentmagnet-Segmente zeigten positive Ergebnisse bei einer Reihe von Gesundheitsbeschwerden, u. a. bei Durchblutungsstörungen, Schmerzzuständen, Narbenbehandlung, verbesserte Regeneration nach Verletzungen /Sportverletzungen, Nacken- und Schulterbereichsprobleme, Arthrose, Lendenwirbelsäulenproblemen, Ischias-, Knie- und Beinbeschwerden, Blutergüssen, Quetschungen, Gelenkverstauchungen, Tennisarm, Spannungskopfschmerzen, Schlafstörungen, Muskelkrämpfen, -verspannungen, Knochenheilungsstörungen etc.

Zudem ist die Magnetfeld-Matratzenauflage so konzipiert, dass diese das gesamte Bettklima, insbesondere die Regulierung von Wärme und Feuchtigkeit, verbessert. Dies wird vorrangig durch die verwendeten Materialien, wie Baumwolle und Schafschurwolle, sowie das erwähnte Baumwoll-Silber-Gewerbe erreicht.

Die Magnetfeld-Matratzenauflage stellt auch ein wichtiges Hilfsmittel zur Neutralisation und/oder Abschwächung von biologisch wirksamen Feldeinflüssen aus den Bereichen der Elektrifizierung, Elektrotechnik und Telekommunikation dar. Durch die Verwendung des elektrisch hochleitfähigen und natürlichen Werkstoffes Silber werden diese Felder innerhalb der Liegefläche über eine spezielle Vorrichtung auf Erdpotential abgeleitet. Gleichzeitig wird auch der Körper über die Matratzenauflage geerdet, was unerwünschte Aufladungen während des Schlafes wirksam verhindert. Zudem wird durch die Erdung des Körpers die körpereigene Polarisation wieder hergestellt.
1. Durch magnetische Ausrichtungen schließen sich Einzelmoleküle zu Ballungen zusammen und werden dadurch intensiver mit Sauerstoff versorgt.
2. Das Magnetfeld führt zu einer höheren Spannung in den Zellen und beschleunigt den lonentransport durch die Zellmembran.
3. Therapeutisch genutzte statische Magnetfelder dämpfen die Übererregung der Nervenfasern, die für die Schmerzempfindung zuständig sind.
4. Durch die pH-Wert-Senkung kommt es zu einer Funktionsoptimierung desselben. Weiße Blutkörperchen können in großer Menge produziert und in den Blut- und Lymphgefäßen ausgeschwemmt werden. Dadurch wird eine direkte Erhöhung der Abwehrkraft erreicht.
5. Ebenso wichtig ist die Erkenntnis, dass durch den Einfluss von magnetischen Feldern ein Druck (sog. Lorenz'sche Kräfte) auf die Gefäßwände ausgeübt wird und diese sich erweitern. Bewiesen wurde dieser Effekt mit einer wissenschaftlichen Studie mit Magnetfolie beim M.I.T. - Massachusetts, Institute of Technology, Cambridge, USA. Die Folge dieser Beeinflussung der Gefäßwende ist eine erhöhte Blutzirkulation mit mehr Sauerstoff- und Nährstofftransport zu den umgebenden Zellstrukturen hin.
6. Schlussendlich ist auch die biologische Komponente des Informationsgehaltes wichtig, welche über natürliche Schwingungsfrequenzen auf den menschlichen Organismus übertragen wird. Da sich Körperflüssigkeiten wie ein bewegtes leitfähiges Medium verhalten, können sie Informationen aufnehmen. Dieser Informationsaustausch im Zellgeschehen ist ein bekanntes Phänomen der Induktion. Jede homöopathische Medikation ist diesem Prinzip unterworfen.

Der nach dem englischen Physiker Hall benannte Hall-Effekt ist eine der wichtigsten durch Magnetfelder ausgelösten und bekannten Effekte. Trifft ein Magnetfeld senkrecht bzw. im Winkel auf eine elektrisch leitfähige Flüssigkeit, so erfolgt eine Ladungstrennung, bspw. in Form einer lonentrennung. Die positiv geladenen lonen (+) werden vom Nordpol (+) des Magnetfeldes abgestoßen, die negativ geladenen lonen (-) angezogen. Beim Südpol (-) vollzieht sich dies in genau umgekehrter Weise. Durch die lonentrennung entsteht dann eine minimale elektrische Spannung.

Unser Blutplasma stellt ebenfalls eine elektrisch leitfähige Flüssigkeit (Elektrolyt) dar und enthält eine Unzahl von lonen, z. B. Natrium (+), Chlor (-). Wenn der Körper auf die Permanentmagnet-Segmente mit unterschiedlicher axialer Magnetisierung (ein Nordpol befindet sich auf der einen Oberfläche und ein Südpol auf der gegenüberliegenden Oberfläche) aufliegt, muss das sich fortbewegende Blutplasma die unterschiedlichen Polfelder durchqueren, wobei die oben beschriebene lonentrennung zwangsweise erfolgt. Der Hall-Effekt tritt also automatisch ein, und es entsteht dabei eine sehr geringe elektrische Spannung. Durch diese Elektrostimulation erfolgt eine geringe Erwärmung, was sich speziell während des Schlafes aufgrund des erhöhten Wärmebedarfes als sehr nützlich erweist.

Maßgeblichen Einfluss an der Weiterentwicklung und wissenschaftlichen Begründung der Magnetfeldtherapie hatte der zweifache amerikanische Nobelpreisträger Linus C. Pauling, der nachwies, dass der eisenhaltige rote Blutfarbstoff Hämoglobin magnetische Eigenschaften besitzt.

Im Folgenden wird die Erfindung von beispielhaften Figuren näher erläutert, die jedoch nicht einschränkend für den Schutzbereich der Erfindung aufzufassen sind.

Es zeigen:
- Figur 1:: ein Teil der erfindungsgemäßen Körperunterlage mit Blick auf dessen Schichtung;
- Figur 2:: die erfindungsgemäße Körperunterlage in einer schematisierten Draufsicht;
- Figur 3:: einen Querschnitt durch die erfindungsgemäße Körperunterlage nach Figur 2,
- Figur 4:: Schnitt durch eine Körperunterlage nach den vorhergehenden Ausführungsbeispielen,
- Figur 5:: Schnitt durch eine weitere Ausführungsform einer Körperunterlage mit einer zusätzlichen Hämatitschicht,
- Figur 6:: Längsschnitt durch eine Körperunterlage nach dem Ausführungsbeispiel der Figur 5,
- Figur 7: eine weitere Anordnung einer Körperunterlage, die in der Art eines Schlafsackes ausgebildet ist,
- Figur 8:: die Seitenansicht der Unterlage nach Figur 7.

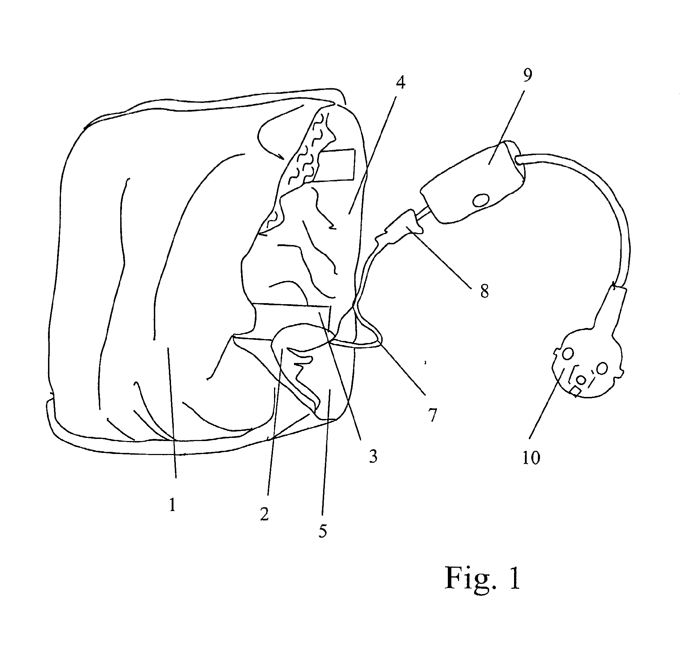

Grundsätzlich ist die erfindungsgemäße Magnetfeld-Matratzenauflage nach Figur 1 optisch aufgebaut wie herkömmliche Matratzenauflagen. Hier der genaue Aufbau:
1.) Die unterste Schicht bzw. Hülle 1 besteht vorzugsweise aus reinem, ungebleichtem Baumwollstoff.
2.) Als nächste Schicht ist eine Isolier- und Wärmeschicht 2 vorgesehen, welche vor allem den Körper vor von unten aufsteigender Kälte schützen soll. Diese Schicht besteht vorzugsweise aus einem Schafschurwoll-Vlies.
3.) Als nächstes kommen die Permanentmagnet-Segmente 3 aus insbesondere Magnetit-Ferriten. Diese bestehen bevorzugt zu 90% aus feinst verteiltem Magnetit-Ferrit-Pulver und zu 10% aus modifiziertem Polyäthylen.
   Vorzugsweise werden die Segmente in Form von Folienstreifen eingesetzt. Für eine Liegefläche von 2000 mm Länge werden 8-10 Folienstreifen mit einer Breite von 300 mm eingesetzt. Die Polarisation ist nun so vorgesehen, dass der Kopf immer in Nordfließrichtung und die Füße in Südfließrichtung der Magnetfelder zu liegen kommen, dies unabhängig von der eigentlichen Schlafrichtung.
   Zwischen den Folienstreifen entsteht, je nach verwendeter Anzahl, ein Abstand. Dadurch entstehen kleine magnetische Wirbelströme, welche nun naturkonform auf den Organismus einwirken können.
   Die Folienstreifen werden an der nächsten Schicht, nämlich dem Baumwoll-Silber-Gewebe, vorzugsweise durch Annähen befestigt.
4.) Als Mittelschicht der Matratzenauflage wird vorzugsweise ein elektrisch leitfähiges, nicht magnetisierbares und atmungsaktives Spezialgewebe 4 aus Baumwolle und reinem Silber verwendet. Dieses Baumwoll-Silber-Gewebe ist bekannt aus der auf die gleiche Anmelderin zurückgehende Offenlegungsschrift DE 199 29 077 A1, deren Offenbarungsgehalt in der vorliegenden Erfindung vollumfänglich umfasst sein soll. Natürlich können auch andere, elektrisch leitfähige und nicht magnetisierbare Materialien eingesetzt werden, wie beispielsweise Aluminium etc., oder Kombinationen und/oder Legierungen aus derartigen elektrisch leitfähigen und nicht magnetisierbaren Materialien.
   Diese Mittelschicht dient zur Abschirmung bzw. Ableitung unerwünschter Energiefelder, insbesondere der elektr. 220 Volt/50-Hz-Wechselfelder und elektrostatischer Aufladungen. Dies wird über einen speziellen Erdungsstecker über das Hausstromnetz erreicht. Es ist technisch auch eine Ableitung über eine Direktverbindung zur Potentialausgleichsschiene oder über die Wasserinstallation (Wasserleitungsrohre) oder Heizungsinstallation (Heizrohre/Heizkörper) möglich, da diese, gemäß den gängigen Vorschriften, ebenfalls auf Erdpotential zu liegen haben.
   Durch die erfindungsgemäße Abschirmmaßnahme mit einem Sack aus einem leitfähigen Silbergewebe, welcher die Magnetsegmente umfasst, wird ein wesentlicher Abschirmeffekt erreicht.
   Es hat sich nämlich herausgestellt, dass das Magnetfeld der erfindungsgemäßen Magnetsegmente gestört wird durch Hochfrequenzeinflüsse von externen Störfrequenzen. Derartige störende elektromagnetische Felder sind z. B. Hochfrequenzfelder, ausgelöst durch Telekommunkationsgeräte, durch Radio, Fernsehen und durch eine Vielzahl von Funkfrequenzen, die sich in der Atmosphäre befinden und die mühelos auch durch entsprechende Stein- und Betonschichten in Häusern hindurchdringen und den Schlafplatz beeinträchtigen.
   Hier setzt die Erfindung ein, die erkannt hat, dass man zunächst diese äußeren, störenden HF-Felder abschirmen muss, damit die in der Abschirmhülle befindlichen Permanentmagnetstreifen ihre magnetische Wirkung auf den darauf liegenden Körper überhaupt entfalten können.
   Dieser Lösungsansatz war bisher noch nicht bekannt. Es ist lediglich bekannt gewesen, die magnetische Wirkung derartiger Permanentmagnete durch Metallplatten oder dergleichen zu verstärken (siehe DE 93 01 830 U1), jedoch ist damit nicht das Problem gelöst, dass man ein gestörtes Magnetfeld - ausgelöst durch Permanentmagnete - erst wieder harmonisieren muss, bevor dieses Magnetfeld auf den Körper der darauf liegenden Person einwirkt.
   Durch diese Maßnahme wird der auf der Magnetfeld-Matratzenauflage liegende Körper ebenfalls auf Erdpotential gebracht. Dies bringt nach bisherigen wissenschaftlichen Erfahrungen eine deutliche Entlastung der auf den Organismus einwirkenden fremden, physikalischen Energiefelder. Vor allem der, durch laufende Aufnahme von Spannungen der elektrischen Felder entstehende, unnatürlichen Aufladung wird dadurch wirksam entgegengewirkt. Zudem begünstigt die Körpererdung die natürliche Polarisation des Körpers (Varga, Becker).
   Mit diesem Spezialgewebe und insbesondere durch die Verwendung des darin verwendeten reinen, elementaren Silbers, wird eine gleichzeitige Abschirmwirkung gegen unerwünschte geobiologische Felder innerhalb der Liegefläche erzielt. Dies haben Messungen mit der Methode der Segmentardiagnostik und biofunktionalen Organometrie gezeigt.
5.) Oberhalb des Spezialgewebes 4 wird eine weitere Schicht zur Regulierung von Körperwärme und Feuchtigkeit, wiederum vorzugsweise ein Schafschurwoll-Vlies 5, verwendet. Dies unterstützt die Vorteile eines trockenwarmen Bettklimas.
6.) Die oberste Schicht (Hülle) 6 besteht vorzugsweise wieder aus reinem, ungebleichten Baumwollstoff.
7.) Das Erdungskabel 7 wird mit dem Baumwoll-Silber-Gewebe mittels eines elektr. leitfähigen Erdungsbandes elektrisch verbunden.
8.) Das Erdungskabel 7 wird über einer Spezialbuchse 8 mit dem Erdungsstecker verbunden.
9.) Eine Kontrollleuchte 9 ist ein integrierter Funktionsteil des Erdungssteckers, der mit einem Längswiderstand (2 kOhm) ausgestattet ist (verhindert Rückströme über die Erdung). Die Kontrollleuchte 9 ist bei einem zusätzlichen Einsatz eines automatischen Netzabkopplers vorteilhaft, da sie die Funktionsweise des Netzabkopplers anzeigt.
10.) Der Schukostecker 10 dient zum Betrieb der Kontrollleuchte 9 und garantiert die ordnungsgemäße Erdung der Magnetfeld-Matratzenauflage über den Schutzleiter. Die Magnetfeld-Matratzenauflage benötigt keine elektrische Energie aus dem Hausstromnetz. Sie arbeitet daher in diesem Sinne absolut stromlos.

Dieser systematische Aufbau berücksichtigt alle Komponenten, dass die der Erfindung zugrundeliegenden und erwünschten Effekte/Anwendungsvorteile eintreten. Es wird dabei auch darauf hingewiesen, dass auch bei den in der Anordnung nicht veränderbaren Magneten immer eine Induktion stattfindet. Dies vor allem durch die bevorzugte Anordnung der Permanentmagnet-Segmente und auch deshalb, weil sich die Lage zwischen der Magnetfeld-Matratzenauflage und dem darauf liegenden Körper ständig verändert; dies allein schon durch die Atembewegungen und den Pulsschlag unseres Körpers sowie durch die Schlafbewegungen. So entsteht ein minimales aber hochwirksames elektrisches Potential im Körper.

Diese Magnetfeld-Matratzenauflage kann auch nach demselben Aufbauschema direkt in eine Matratze oder ein Schlafsystem integriert werden.

In einer weiteren und angepassten Anwendungsform soll diese Magnetfeld-Auflage z. B. auch in Form von Sitzauflagen an Arbeitsplätzen oder als Autositzauflage Verwendung finden. Ebenfalls ist ein Einsatz in anderen Transportmitteln wie Bus, Bahn, Schiff oder Flugzeug, möglich; ebenso im Kur-, Wellness-, Krankenhaus-, Ärzte- und Therapiebereich. Als Spezialauflage ist auch eine vorteilhafte Anwendung als Unterlage von Magnetfeldmatten für pulsierende Magnetfelder gedacht, ebenfalls als direkte oder indirekte Ergänzung zu jeder anderen Therapieform.

Die Magnetfeld-Matratzenauflage ist ebenfalls mittels des verwendeten reinen Silbers und der Permanentmagnet-Segmente zur Heilmittel-Informationsübertragung geeignet (Neue Homoöpathie). Wie bei einer Scheckkarte Informationen auf dem Magnetstreifen festgehalten werden, erfolgt bei der Magnetfeld-Matratzenauflage die Abspeicherung von Heilinformationen im Transferenzverfahren über einen Radionic Computer. Dadurch lassen sich x-beliebige Arzneiinformationen auf den Trägerelementen der Permanentmagnet-Segmente und/oder des reinen, elementaren Silbers speichern. Diese werden dann bedarfsweise vom Organismus, respektive von den informationsresonanten Zellen aufgenommen. Der Vorteil dieser Anwendungsform liegt in der nächtlichen Langzeit-Therapie. Zudem entfallen viele bekannte Nebenwirkungen und Organbelastungen von herkömmlichen Medikamenten.

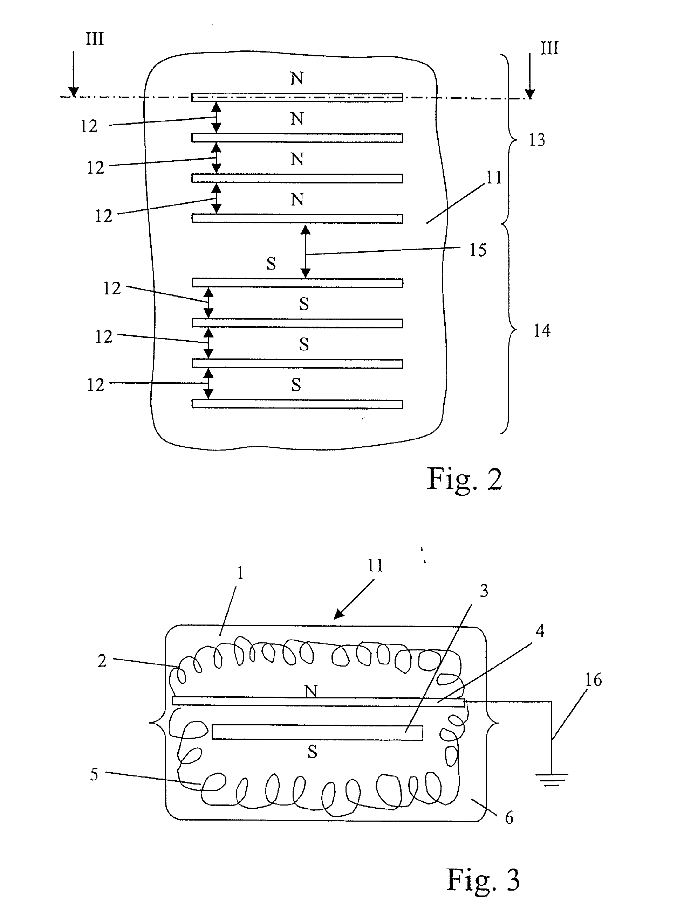

Figur 2 zeigt das Magnetfeldunterbett, welches in seiner Liegefläche 11 im Abstand 12 parallel zueinander Permanentmagnetstreifen 3 angeordnet hat, von denen die im oberen Teil 13 der Liegefläche 11 angeordneten Magnetstreifen 3 an ihrer Oberseite jeweils auf Nordpol polarisiert sind, während die an der Unterseite 14 angeordneten Magnetstreifen 3 an ihrer Oberseite jeweils den Südpol tragen.

Im Mittenbereich der Liegefläche 11 ist ein Abstand 15 zwischen den aneinander angrenzenden Magnetstreifen 3 vorhanden, um die Ausbildung entsprechender Wirbelströme oder Beeinträchtigung der Entstörwirkung zu mindern.
Ferner liegt die Kenntnis zugrunde, dass man oberhalb der Magnetstreifen 3 eine, insbesondere der Entstörung von elektrostatischen Feldern dienende, Silbergewebeabschirmung 4 einsetzt, die in an sich bekannter Weise mit einer Erdung 7-10 verbunden ist.
Insgesamt ergibt sich der Vorteil, dass zunächst mit der Abschirmung insbesondere elektrostatischer Felder eine Beruhigung des auf den Schläfer einwirkenden Feldes in der Weise erreicht wird, dass derartige elektrostatische Felder bereits schon wirksam unterbunden werden. Es werden dann lediglich heilsame Magnetfelder durch die Permanentmagnet-Anordnung auf den Schläfer erzeugt, was zu einer wesentlichen Verbesserung der Körperfunktionen führt.

Figur 3 zeigt die Einbettung der Permanentmagnet-Anordnung innerhalb der Liegefläche 11 der Magnetfeld-Matratzenauflage im Querschnitt, entlang der Schnittlinie III-III der Figur 2.

Hier ist zu sehen, dass die Schicht des Silbergewebes 4 zur Ableitung induzierter Ströme, die durch unerwünschte fremder Energiefelder erzeugt werden, mittels der Erdung 16 geleitet werden und somit für den Nutzer unschädlich gemacht werden.

Die Schicht des Silbergewebes 4 ist hierbei der Liegefläche 11 für den Nutzer zugewandt. Darunter liegen die Magnetstreifen 3, welche nahezu die gesamte Breite der Matratzenauflage überspannen.

Oberhalb der Schicht des Silbergewebes 4 und unterhalb der Magnetstreifen 3 sind wärmeisolierende Schichten 2, 5 eingebracht sowie die gesamte Anordnung umhüllende Deckschichten 1, 6 aus Baumwolle.

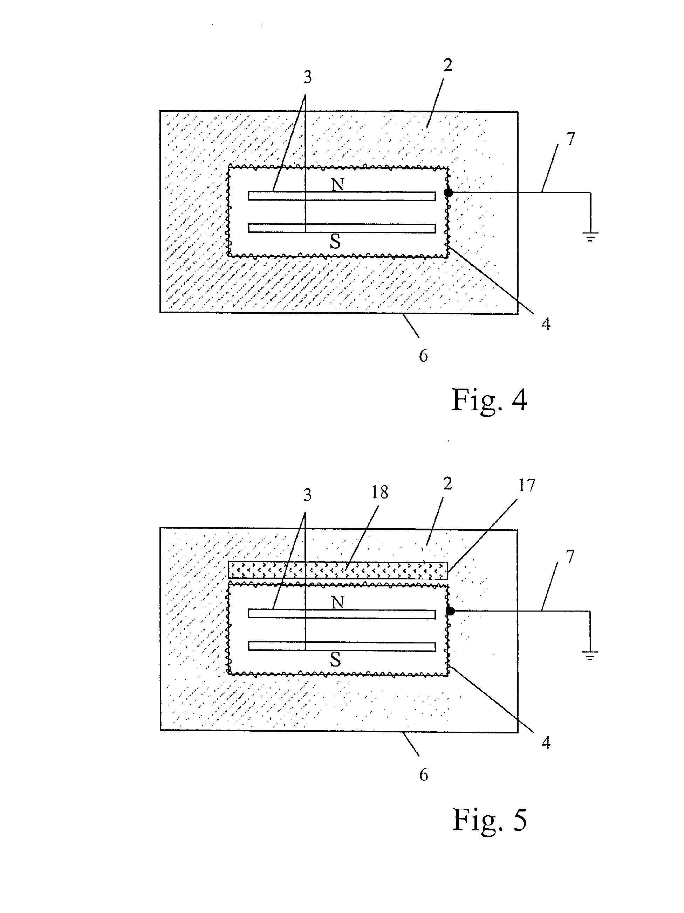

Figur 4 zeigt nochmals die Anordnung nach den Figuren 1 bis 3 im Schnitt, wobei erkennbar ist, dass die Körperunterlage gebildet ist aus einer Hülle 6 aus Baumwolle, in der ein Schafschurvlies 2 als Polsterung angeordnet ist.

In einem sackartigen Gebilde aus dem HF-Felder abschirmenden Spezialgewebe 4 befinden sich nun im Abstand und parallel zueinander angeordnet die Magnetstreifen. Der besseren Übersichtlichkeit halber wurden diese Magnetstreifen weit auseinander gezeichnet. In Wirklichkeit handelt es sich um dünne Folien, wobei die Oberseite der Folie beispielsweise als Nordpol und die Unterseite der Folie als Südpol polarisiert ist.

Diese Folienstreifen sind parallel und in gegenseitigem Abstand zueinander senkrecht zur Körperlängsachse in dem Innenraum des Abschirmsackes angeordnet.

Die Figur 5 zeigt in Erweiterung des Ausführungsbeispieles nach den Figuren 1 bis 4, dass noch eine zusätzliche Hämatitschicht 18 verwendet werden kann.

In einem Schlauchgewebe 17 ist ein feinkörniges Hämatitkorn angeordnet. Es handelt sich hierbei um das körnige Verwitterungsprodukt des Magnetiten.

Dieses Hämatitkorn hat die besondere Eigenschaft, paramagnetisch zu sein. Es wird dadurch durch das darunter liegende statische Magnetfeld durch die Permanentmagneten 3 auch magnetisch und dadurch wird die biologische Wirksamkeit der Permanentmagneten 3 wesentlich verstärkt.

Bei einer Korngröße von 0 bis 0,6 mm hat die Hämatitschicht 18 eine Schichtdicke von z.B. 5 bis 10 mm, wobei diese Hämatitschicht in dem Schlauchgewebe 17 rieselfähig angeordnet ist. Das Schlauchgewebe ist so dicht, dass das Hämatitkorn nicht herausrieseln kann.

Es ist zusätzlich luftdurchlässig und feuchtigkeitsabsorbierend, so dass auch noch eine Verbesserung des physikalischen Schlafklimas erreicht wird.

Die gesamte Hämatitschicht 18 erstreckt sich vorzugsweise über die gesamte Fläche der Unterlage und bedeckt diese demgemäss vollflächig.

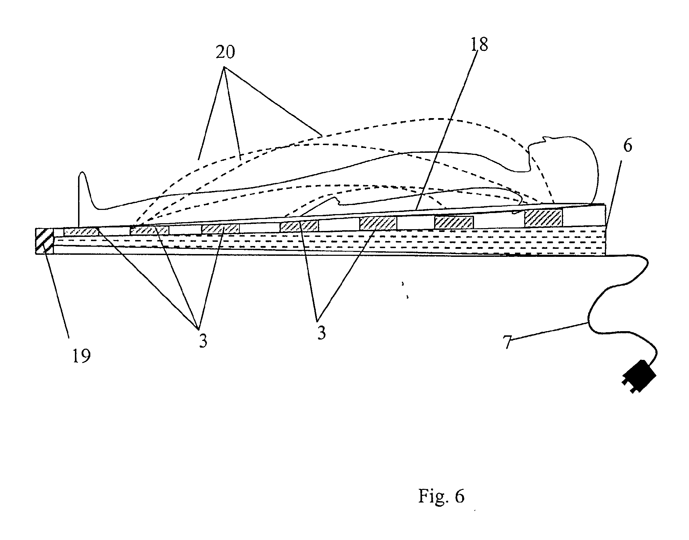

Figur 6 zeigt eine vollflächige Auflage einer Hämatitschicht, wobei schematisiert die darunter liegenden, parallel und in gegenseitigem Abstand zueinander angeordneten Permanentmagnetsegmente 3 vorgesehen sind.

Es ist die abschirmende Hülle aus dem Spezialgewebe 4 nur angedeutet. Es gilt jedoch die Erläuterung nach Figur 5.

Bei Figur 6 ist auch noch wichtig, dass das Erdungskabel 7 zwar am Kopfende der Unterlage herausgeführt wird, dass dieses aber elektrisch leitfähig mittels eines Kabels bis zum Fußende hindurchgeführt wird und dort elektrisch leitfähig mit einer Erdungsleiste 19 verbunden ist. Diese Erdungsleiste ist elektrisch leitfähig mit dem fußseitigen Ende des Abschirmsackes aus dem Spezialgewebe 4 verbunden.

Es hat sich nämlich als wichtig herausgestellt, dass lediglich an der Fußseite geerdet wird, weil die biologische Polarität so ist, dass der Kopf im Pluspotential liegt und damit der Fuß geerdet ist, wodurch es zu einem günstigen Potentialausgleich kommt.

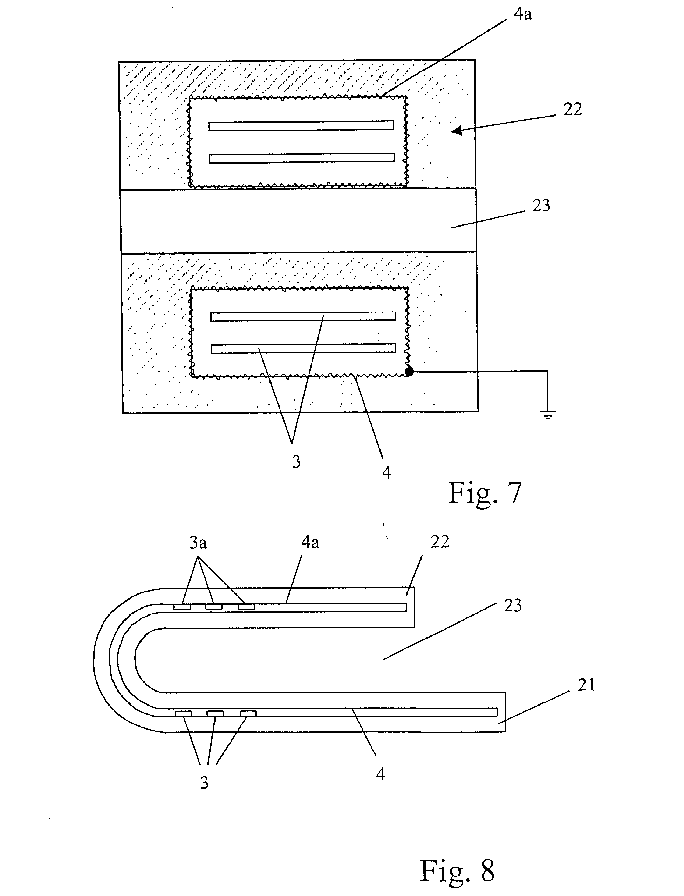

Die Figuren 7 und 8 zeigen als weiteres Ausführungsbeispiel, dass nach einer oder mehreren der vorher genannten Ausführungsbeispiele der Figuren 1 bis 5 auch die Möglichkeit besteht, statt der Unterlage ein komplettes Gebilde in der Art eines Schlafsackes zu schaffen.

Hierbei ist die Unterlage 21 in einer der Ausführungsformen nach Figur 1 bis 5 ausgebildet und setzt sich in Form eines Oberteils 22 in der Art eines Schlafsackes fort. Dies ist in Figur 8 anhand des Ausführungsbeispieles nach Figur 4 dargestellt. Dort ist erkennbar, dass der Abschirmsack mit dem Spezialgewebe 4 am Fußteil hochgeführt ist und in dem Oberteil 22 als Spezialgewebe 4a einen weiteren Abschirmsack bildet, in dem ebenfalls die Permanentmagnetsegmente 3a angeordnet sind.

Die Anordnung nach Figur 4 kann also sowohl im Unterteil 21 als auch im Oberteil 22 vorhanden sein und auch in beiden Teilen, wie dies die Figur 8 zeigt.

Vorteil ist, dass - wenn man den Abschirmsack im Oberteil 22 unmittelbar mit dem Abschirmsack im Unterteil 21 verbindet - man nur eine einzige elektrische Abschirmung über das Erdungskabel 7 benötigt.

Im Bereich des Aufnahmeraumes 23, in dem der menschliche Körper liegt, kommt es dann zur Ausbildung eines faraday'schen Käfigs, so dass der Körper völlig von außen liegenden Strahlungen und Magnetfeldern freigehalten wird. Es wirken dann nur noch die Magnetfelder im Ober- und Unterteil 21,22, wie sie erfindungsgemäß durch die folienförmigen Permanentmagnetsegmente 3 vorgesehen sind.

Es hat sich im übrigen gezeigt, dass die Anordnung von derartigen Permanentmagnetsegmenten 3, 3a mit einem Abschirmsack aus dem Spezialgewebe 4 bei der Auflage auf einer Federkernmatratze zu besonders günstigen therapeutischen Effekten führt.

Aufgrund der Magnetisierbarkeit des Drahtgewebes durch das Erdmagnetfeld ist das von Haus aus vorhandene Magnetfeld einer Federkernmatratze schon gestört. Durch die Auflage der Permanentmagnetsegmente 3 in Verbindung mit dem Abschirmsack 4 und/oder 4a wird damit das natürlich vorhandene Feld der Federkernmatratze harmonisiert und praktisch beseitigt. Die Störungen, welche die Federkerne durch das Erdmagnetfeld erhalten haben, werden also durch die darauf liegenden Permanentmagnetsegmente harmonisiert und beseitigt.

Außerdem ist es bekannt, dass die aus elektrisch leitfähigem Draht bestehenden Drahtfedern der Federkernmatratze an elektrische niederfrequente Magnetfelder einkoppeln und sozusagen als Antenne wirken, welche diese Strahlen weitergeben.

Durch die Auflage eines Abschirmsackes mit dem Spezialgewebe 4 wird dadurch auch die Weitergabe derartiger Störungen auf den Körper vermieden.

Die Erfindung betrifft also insbesondere eine Magnetfeld-Köperunterlage, welche mindestens einen, bevorzugt jedoch eine Vielzahl von untereinander beabstandeten, Permanentmagneten mit Nord- und Südpol enthält und mindestens eine, mindestens teilweise elektrisch leitfähige, jedoch nicht magnetisierbare und elektrisch erdbare, Schicht zur Abschirmung unerwünschter fremder Energiefelder, auf welcher die Permanentmagnete in Folienform aufgebracht sind. Umhüllt wird diese Anordnung von einem wärmedämmenden Schafschurwoll-Vlies und einem äußeren Baumwollstoff. Durch Abschirmung schädlicher fremder Energiefelder und Einkopplung von erwünschten, heilsamen Magnetfeldern in den Körper, die dem natürlichen Erdmagnetfeld in gesteigerter Form entsprechen, wird ein prophylaktischer und therapeutischer Effekt erzielt, der das Schlafverhalten, die Schlafqualität sowie das energetische Gleichgewicht der Köperzellen positiv beeinflussen.

### Bildlegende

- 1: Hülle aus Baumwolle (unten)
- 2: Schafschurwoll-Vlies
- 3: Permanentmagnet-Segment 3a
- 4: Spezialgewebe Baumwoll-Silber 4a
- 5: Schafschurwoll-Vlies
- 6: Hülle aus Baumwolle (oben)
- 7: Erdungskabel
- 8: Erdungsbuchsen-Verbindung
- 9: Kontrollleuchte (für Betrieb Netzfreischalter)
- 10: Schukostecker
- 11: Liegefläche
- 12: Abstand Magnetstreifen N-N und S-S
- 13: Oberteil
- 14: Unterteil
- 15: Abstand Magnetstreifen N-S
- 16: Erdung von 4
- 17: Schlauchgewebe
- 18: Hämatitschicht
- 19: Erdungsleiste
- 20: Magnetfeld
- 21: Unterteil
- 22: Oberteil
- 23: Aufnahmeraum

## Patentansprüche

1. Auflage oder Unterlage mit einer Vorrichtung zur Erzeugung eines Permanent-Magnetfeldes auf den darauf liegenden menschlichen Körper, mit flachen, polarisierten folienförmigen Magnetstreifen, die im gegenseitigen Abstand und parallel zueinander in der Auflage angeordnet sind, **dadurch gekennzeichnet, dass** die das Magnetfeld erzeugende Anordnung in einem Abschirmsack angeordnet ist, der aus mindestens einer, mindestens teilweise elektrisch leitfähigen, jedoch nicht magnetisierbare Schicht (4) besteht, die geerdet ist.

2. Auflage oder Unterlagenach Anspruch 1, **dadurch gekennzeichnet, dass** der Abstand (12) zwischen den jeweils gleichgerichteten Nord- und Süd-Permanentmagneten (3) etwa konstant ist.

3. Auflage oder Unterlagenach einem der Ansprüche 1 oder 2, **dadurch gekennzeichnet, dass** alle Nordpole der Permanentmagnete (3) und anschließend alle Südpole hintereinanderfolgend angeordnet sind und zwischen den Nordpolen und den Südpolen ein Abstand (15) besteht, der dem Abstand (12) zwischen den übrigen, jeweils gleichgerichteten Nord- oder Süd-Permanentmagneten (3) nicht entsprechen muss.

4. Auflage oder Unterlagenach einem der Ansprüche 1 bis 3, **dadurch gekennzeichnet, dass** die Schicht (4) des Abschirmsackes ein Spezialgewebe ist, welches ein Material aus reinem Silber enthält.

5. Auflage oder Unterlagenach Anspruch 4, **dadurch gekennzeichnet, dass** das Spezialgewebe dem der Offenlegungsschrift DE 199 29 077 A1 entspricht.

6. Auflage oder Unterlagenach Anspruch 1 oder 4 oder 5, **dadurch gekennzeichnet, dass** die Schicht (4) einen Schutzkontaktstecker (10) aufweist, welcher eine elektrische Erdung (16) über die elektrische Netzversorgung der Haushalte bewirkt.

7. Auflage oder Unterlagenach einem der Ansprüche 1 bis 6, **dadurch gekennzeichnet, dass** die Magnetfeld-Köperunterlage als Matratzenauflage, Schlafdecke oder Sitzkissen ausgebildet ist oder aber ein textiles Bekleidungsstück bildet.

8. Auflage oder Unterlagenach einem der Ansprüche 1 bis 7, **dadurch gekennzeichnet, dass** der mindestens eine Permanentmagnet (3) derart auf der Magnetfeld-Köperunterlage ausgerichtet ist, dass die Nordpol-Südpol-Ausrichtung im wesentlichen in der Längsachse des Körper des Nutzers liegt.

9. Auflage oder Unterlagenach einem der Ansprüche 1 bis 8, **dadurch gekennzeichnet, dass** der Nordpol des mindestens einen Permanentmagneten (3) auf der Kopfseite (13) des Nutzers und der Südpol auf der Fußseite (14) liegt.

10. Auflage oder Unterlagenach einem der Ansprüche 1 bis 9, **dadurch gekennzeichnet, dass** der mindestens eine Permanentmagnet (3) sich im wesentlichen über die gesamte Breite der Magnetfeld-Köperunterlage erstreckt.

11. Auflage oder Unterlagenach einem der Ansprüche 1 bis 10, **dadurch gekennzeichnet, dass** die Stärke des Magnetfeldes des mindestens einen Permanentmagneten (3) etwa 10 mal größer ist, als das derzeitig mittlere, natürliche Erdmagnetfeld.

12. Auflage oder Unterlagenach einem der Ansprüche 1 bis 11, **dadurch gekennzeichnet, dass** in die folienförmigen Permanentstreifen eine zusätzliche digitale Information eingeschrieben ist.

13. Auflage oder Unterlagenach einem der Ansprüche 1 bis 12, **dadurch gekennzeichnet, dass** im Innenraum des Abschimsackes oberhalb der Ebene mit den Permanentmgnetstreifen eine weitere Schicht (18) angeordnet ist, die aus einem in körniger Form vorliegenden Mineralstoff (Hämatit) besteht.

14. Auflage oder Unterlagenach Anspruch 13, **dadurch gekennzeichnet, dass** die Schicht (18) in einem Schlauchgewebe (17) angeordnet ist.

15. Auflage oder Unterlage nach einem der Ansprüche 1 bis 14, **dadurch gekennzeichnet, dass** das Erdungskabel (7) am Kopfende der Unterlage herausgeführt und mittels eines Kabels bis zum Fußende hindurchgeführt ist und dort elektrisch leitfähig mit einer Erdungsleiste 19 verbunden is, die mit der Schicht(4) aus Silbergewebe kontaktiert ist.

16. Auflage nach einem der Ansprüche 1 bis 15, **dadurch gekennzeichnet, dass** die Auflage oder Unterlage in Form eines Schlafsackes ausgebildet ist und die vorbeschriebenen und beanspruchten Elemente der Auf- oder Unterlage (21) als Oberteil (22) den menschlichen Körper von der Oberseite her umhüllen.

17. Auflage oder Unterlage nach einem der Ansprüche 1 bis 16, **dadurch gekennzeichnet, dass** die Auf- oder Unterlage (21,22) zur Harmonisierung des gestörten Magnetfeldes einer Federkernmatratze dient.
